# EUROPÄISCHE PATENTSCHRIFT

(11) **EP 0 255 082 B1**
(45) Veröffentlichungstag und Bekanntmachung des Hinweises auf die Patenterteilung: **01.12.1993**
(21) Anmeldenummer: 87110847.8
(22) Anmeldetag: 27.07.1987
(51) Int. Cl.: C07K 5/02, C07K 5/06, C07D 233/64, C07K 1/00, A61K 37/64, A61K 31/415

(54) **Renin-hemmende Di- und Tripeptide, Verfahren zu deren Herstellung, diese enthaltende Mittel und ihre Verwendung**
Renin-inhibiting di- and tripeptides, methods for their production, drugs containing them, and their use
Di- et tripeptides inhibant la rénine, méthodes de production, médicaments les contenant et leur application

(30) Priorität: 30.07.1986 DE 3625687; 16.08.1986 DE 3627877
(43) Veröffentlichungstag der Anmeldung: 03.02.1988
(73) Patentinhaber: HOECHST AKTIENGESELLSCHAFT, 65926 Frankfurt am Main (DE)
(72) Erfinder: Wagner, Adalbert, Dr., D-6238 Hofheim am Taunus (DE); Kleemann, Heinz-Werner, Dr., D-6092 Kelsterbach (DE); Ruppert, Dieter, Dr., D-6233 Kelkheim Taunus (DE); Schölkens, Bernward, Dr., D-6233 Kelkheim Taunus (DE)

(56) Entgegenhaltungen:
- EP-A- 0 128 762
- EP-A- 0 163 237
- EP-A- 0 172 346
- EP-A- 0 173 481
- EP-A- 0 189 203
- EP-A- 0 202 577
- Chemistry Letters, no. 7, July 1985 pages 1041-1044, The Chemical Society of Japan; R Matsueda et al.: "Short chain peptide inhibitors of human renin"
- Breipohl G et al., Topics in Medicinal Chemistry 102-127 (1988, Royal Society Chemistry, London)

## Beschreibung

Aus den EP-A-152 255, EP-A-155 809, EP-A-163 237, EP-A-172 346, EP-A-172 347 und EP-A-179 352 sind Di- und Tripeptidderivate und deren Verwendung als Renin-Inhibitoren bekannt.

Es wurden neue Peptidderivate gefunden, die in vitro und in vivo hochwirksam das Enzym Renin hemmen.

Die Erfindung betrifft Verbindungen der Formel I 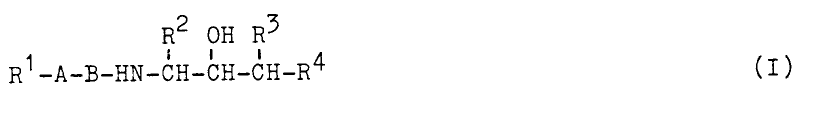
in welcher
- R¹: einen Rest der Formel II bedeutet,

R^{a}-W- (II)

worin W für -CO-, -O-CO-, -SO₂- oder -NH-CO- steht und R^{a} Wasserstoff, (C₁-C₁₀)-Alkyl, das gegebenenfalls ein- oder zweifach ungesättigt ist und das gegebenenfalls durch bis zu 3 gleiche oder verschiedene Reste aus der Reihe Hydroxy, (C₁-C₇)-Alkoxy, (C₁-C₇)-Alkanoyloxy, Carboxy, (C₁-C₇)-Alkoxycarbonyl, Cl, Br, Amino, (C₁-C₇)-Alkylamino, Di-(C₁-C₇)-alkylamino, (C₁-C₅)-Alkoxycarbonylamino, (C₇-C₁₅)-Aralkoxycarbonylamino und 9-Fluorenylmethyloxycarbonylamino substituiert ist, (C₃-C₈)-Cycloalkyl, (C₃-C₈)-Cycloalkyl-(C₁-C₆)-alkyl, (C₆-C₁₄)-Aryl, das gegebenfalls durch einen oder zwei gleiche oder verschiedene Reste aus der Reihe F, Cl, Br, J, Hydroxy, (C₁-C₇)-Alkoxy, (C₁-C₇)-Alkyl, (C₁-C₇)-Alkoxycarbonyl, Amino, gegebenenfalls mit bis zu 2 Halogen substituiertes Anilino und Trifluormethyl substituiert ist, (C₆-C₁₄)-Aryl-(C₁-C₆)-alkyl, worin der Arylteil gegebenenenfalls durch einen oder zwei gleiche oder verschiedene Reste aus der Reihe F, Cl, Br, J, Hydroxy, (C₁-C₇)-Alkoxy, (C₁-C₇)-Alkyl, (C₁-C₇)-Alkoxycarbonyl, Amino, (C₁-C₇)-Alkylamino, Di-(C₁-C₇)-alkylamino, Carboxy, Carboxymethoxy, Amino-(C₁-C₇)-alkyl, (C₁-C₇)-Alkylamino-(C₁-C₇)-alkyl, Di-(C₁-C₇)-alkylamino-(C₁-C₇)-alkyl, (C₁-C₇)-Alkoxycarbonylmethoxy, Carbamoyl, Sulfamoyl, (C₁-C₇)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 S- oder O-Atom auch als Ringglieder steht, der gegebenenfalls durch einen oder zwei gleiche oder verschiedene Reste aus der Reihe F, Cl, Br, Hydroxy, (C₁-C₇)-Alkoxy, (C₁-C₇)-Alkyl, (C₁-C₇)-Alkoxycarbonyl, Amino oder Trifluormethyl substituiert ist,
- R⁴: einen Rest der Formel IV bedeutet,

- X - (CH₂)_{n'} - R⁷ (IV)
- A: einen N-terminal mit R¹ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,
- B: einen Rest einer Aminosäure, wie unter A definiert, bedeutet,
- R²: Wasserstoff, (C₁-C₁₀)-Alkyl, (C₄-C₇)-Cycloalkyl, (C₄-C₇)-Cycloalkyl-(C₁-C₄)-alkyl, (C₆-C₁₄)-Aryl oder (C₆-C₁₄)-Aryl-(C₁-C₄)-alkyl bedeutet und
- R³: Wasserstoff, (C₁-C₁₀)-Alkyl, (C₆-C₁₄)-Aryl oder (C₆-C₁₄)-Aryl-(C₁-C₄)-alkyl bedeutet,
- X: abwesend ist,
- n': = 0, 1, 2, 3 oder 4 sein kann,
- R⁷: Heteroaryl, das auch teilweise hydriert sein kann
sowie deren physiologisch verträglichen Salze, wobei die in EP-A 128762 beschriebenen Verbindungen, bei denen R²=butyl, R³=H und R⁷=4-imidazol ist, ausgenommen sind.

Die durch R², Hydroxy und R³ substituierten C-Atome können jeweils die R-, S- oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

(C₆-C₁₄)-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit (C₁-C₆)-Alkyl verknüpften unsubstituierten oder substituierten (C₆-C₁₄)-Aryl-Rest, wie z.B. Benzyl, α- und β-Naphthylmethyl, Halobenzyl und Alkoxybenzyl, wobei Aralkyl jedoch nicht auf die genannten Reste beschränkt wäre.

Unter einem Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom als Ringglieder werden Reste von Heteroaromaten verstanden, wie sie beispielsweise in Katritzky, Lagowski, Chemie der Heterocyclen, Berlin, Heidelberg 1968, Seite 3 - 5 definiert sind. Der Heteroaromaten-Rest kann durch einen, zwei oder drei, vorzugsweise einen oder zwei gleiche oder verschiedene Reste aus der Reihe F, Cl, Br, Hydroxy, (C₁-C₇)-Alkoxy, (C₁-C₇)-Alkyl, (C₁-C₇)-Alkoxycarbonyl, Amino oder Trifluormethyl substituiert sein. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für Heteroaryl abgeleitete Reste, wie z.B. ganz oder teilweise hydriertes Heteroaryl, Heteroaryloxy, Heteroaryl und Heteroaryl-alkyl.

Die Aminosäuren A und B in Formel I sind durch eine Amidbindung miteinander verknüpft, es handelt sich um natürliche oder nichtnatürliche α-Aminosäuren der L-, D-oder D,L-Konfiguration, vorzugsweise der L-Konfiguration. Entsprechendes gilt für Aminosäuren oder deren Derivate als Reste R⁴.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in denen die Reste wie folgt definiert sind:
- R¹: ist vorzugsweise (C₁-C₁₁)-Alkanoyl, wie n-Decanoyl, Formyl, Acetyl, Pivaloyl, Isovaleryl oder Isobutyryl, gegebenenfalls geschützes Amino-(C₁-C₁₁)-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl, Di-(C₁-C₇)-alkylamino-(C₂-C₁₁)-alkanoyl, wie Dimethylaminoacetyl, (C₄-C₉)-Cycloalkylcarbonyl, wie Cyclopropylcarbonyl, Cyclobutylcarbonyl, Cyclopentylcarbonyl oder Cyclohexylcarbonyl, (C₆-C₁₀)-Aryl-(C₂-C₁₁)-alkanoyl, wie Phenylacetyl, Phenylpropanoyl oder Phenylbutanoyl, 2-(2,6-Dichloroanilino)-phenylacetyl, 2-(N-Benzyl-2,6-dichloranilino)-phenylacetyl, gegebenenfalls durch Halogen, (C₁-C₇)-Alkyl, (C₁-C₇)-Alkoxy oder (C₁-C₇)-Alkoxycarbonyl substituiertes Benzoyl, wie 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzolsulfonyl, (C₁-C₁₀)-Alkoxycarbonyl, wie Methoxycarbonyl, Ethoxycarbonyl oder tert.-Butoxycarbonyl, durch Halogen substituiertes (C₁-C₁₀)-Alkoxycarbonyl, wie 2,2,2-Trichlorethoxycarbonyl oder 1,1-Dimethyl-2,2,2-trichlorethoxycarbonyl, (C₆-C₁₄)-Aryl-(C₁-C₆)-alkoxycarbonyl, wie Benzyloxycarbonyl oder 9-Fluorenylmethylcarbonyl.
- R²: ist vorzugsweise Isobutyl, Benzyl oder Cyclohexylmethyl,
- R³: vorzugsweise Wasserstoff, Isopropyl oder Isobutyl,
- R⁴: vorzugsweise wie oben definiert,.

Bevorzugte Aminosäuren, die für die Reste A und B in Frage kommen, sind Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, 1,2,3,4-Tetrahydroisochinolin-3carbonsäure, Homophenylalanin, 2-Amino-4-(2-thienyl)buttersäure, (Z)-Dehydrophenylalanin oder (E)-Dehydrophenylalanin. Daneben bedeutet A vorzugsweise einen Rest der Formel III wie unter (b₁) beschrieben.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln VIIa - VII d:

R¹-OH (VII a)

R¹-A-OH (VII b)

R¹-A-B-OH (VII c)

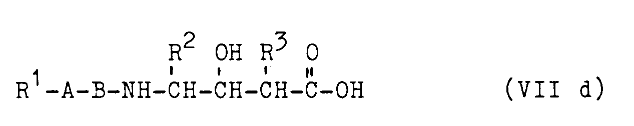

Fragmente einer Verbindung der Formel I mit einer endständigen Aminogruppe besitzen die nachstehenden Formeln VIII a - VIII d:

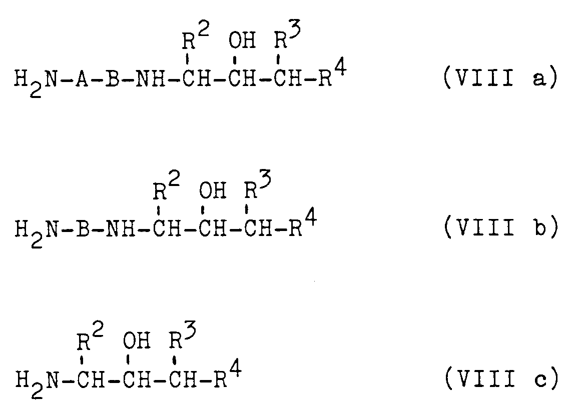

NH₂-R⁸ (VIII d)

Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen:
Aktivestermethode mit N-Hydroxy-succinimid als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Die Herstellung der als Ausgangsverbindungen verwendeten optisch aktiven Amine der Formel IX 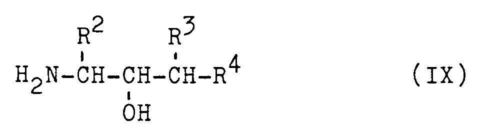
worin R², R³ und R⁴ wie oben definiert sind, erfolgt ausgehend von optisch aktiven α-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Heteroarylalkyl-Baustein gekuppelt wird und nach Abspaltung der N-Schutzgruppe Aminoalkohole der Formel IX ergibt. Man erhält Diastereomerengemische bezüglich des OH-tragenden Zentrums, die in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H.S. Mosher et. al., J. org. Chem. 34, 2543 (1969)).

Die Herstellung N-geschützter Aminosäurealdehyde erfolgt nach B. Castro et al. (Synthesis 1983, 676).

Die Aldol-analoge Addition an N-geschützte Aminosäurealdehyde (bevorzugterweise N-tert.-Butoxycarbonyl-und Benzyloxycarbonyl Schutzgruppen) erfolgt in einem gegenüber Basen inerten Lösungsmittel, wie Ether, THF, Toluol, DMF, DMSO oder Dimethoxyethan.

Als Basen zur Deprotonierung der Heteroarylalkyl-Komponente können Alkalimetallalkoholate, wie Kalium-0-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Aminosäuren A oder B anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin 1 ab. Dieses wird durch das "angiotensin converting enzyme" (ACE) in Angiotensin 2 überführt. Angiotension 2 spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin 1, was eine verminderte Bildung von Angiotensin 2 zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin 1 in verschiedenen Systemen (Humanplasma, Schweine-Renin) gemessen. Hierzu wird z.B. Humanplasma, welches sowohl Renin als auch Angiotensinogen enthält, bei 37°C mit der zu testenden Verbindung inkubiert. Anschließend wird die Konzentration des während der Inkubation gebildeten Angiotensin 1 mit einem Radioimmunoassay gemessen. Die in der vorliegenden Erfindung beschriebenen Verbindungen der allgemeinen Formel I zeigen in verwendeten in-vitro-Tests Hemmwirkungen bei Konzentrationen von etwa 10⁻⁵ bis 10⁻¹⁰ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreichert und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam. Die in der vorliegenden Erfindung beschriebenen Verbindungen der allgemeinen Formel I können als Antihypertensiva, sowie zur Behandlung der Herzinsuffizienz verwendet werden.

Die Erfindung betrifft daher auch die Verwendung von Verbindungen der Formel I als Heilmittel und pharmazeutische Präparate, die diese Verbindungen enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

### Verzeichnis der verwendeten Abkürzungen:

- Ac: Acetyl
- ACHPA: [3S,4S]-4-Amino-3-hydroxy-5-cyclohexylpentansäure
- BOC: tert.-Butoxycarbonyl
- DC: Dünnschichtchromatographie
- DCC: Dicyclohexylcarbodiimid
- DNP: 2,4-Dinitrophenyl
- DMF: Dimethylformamid
- DMSO: Dimethylsulfoxid
- EE: Essigsäureethylester
- Etoc: Ethoxycarbonyl
- FAB: Fast atom bombardment
- H: Hexan
- HOBt: 1-Hydroxybenzotriazol
- Iva: Isovaleryl
- M: Molekularpeak
- MeOH: Methanol
- MS: Massenspektrum
- MTB: Methyl-tert.-butylether
- R.T.: Raumtemperatur
- Schmp.: Schmelzpunkt
- Sta: [3S,4S]-4-Amino-3-hydroxy-6-methyl-heptansäure
- Thi: β-2-Thienylalanin
- THF: Tetrahydrofuran
- Z: Benzyloxycarbonyl

Die sonstigen für Aminosäuren verwendeten Abkürzungen entsprechen dem in der Peptidchemie üblichem drei Buchstaben-Code wie er z.B. in Europ. J. Biochem. 138, 9 - 37 (1984) beschrieben ist. Falls nicht ausdrücklich anders angegeben, handelt es sich immer um Aminosäuren der L-Konfiguration.

Die Bezeichnung "R" wie z.B. in 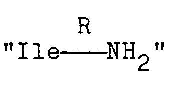
steht für eine Amidbindung, in der die Carbonylgruppe durch eine Methylengruppe ersetzt ist.

Die nachstehenden Beispiele dienen zur Erläuterung der vorliegenden Erfindung, ohne daß diese darauf beschränkt wäre.

### Referenzbeispiel 1

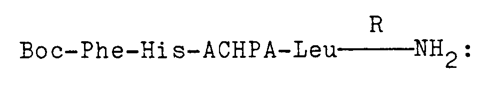

80 mg 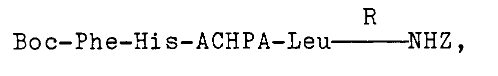
gelöst in 10 ml Methanol, werden mit 50 mg Pd auf Aktivkohle (10 %) als Katalysator mit H₂ unter Normaldruck hydriert. Nach 6 h bei R.T. wird vom Katalysator über Kieselgur abfiltriert. Man engt das Filtrat ein und lyophilisiert, wobei man ein farbloses Pulver erhält.

R_{f} = (EE/Methanol 1:1) = 0,15; MS (FAB): 698 (M+1)
a) 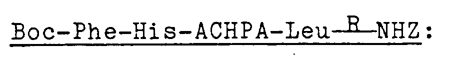 230 mg 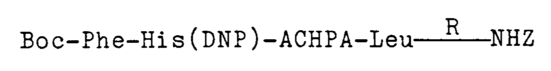 werden in 5 ml DMF mit 0,5 ml Thiophenol 3 h bei R.T. gerührt. Man engt im Vakuum ein und digeriert 3 mal mit Diisopropylether. Nach Chromatographie an Kieselgel (Laufmittel EE/Methanol 15:1) und Einengen der produkthaltigen Fraktionen erhält man ein farbloses Pulver.
   R_{f} (EE/MeOH 5:1) = 0,5 ; MS (FAB) : 832 (M+1)
b) 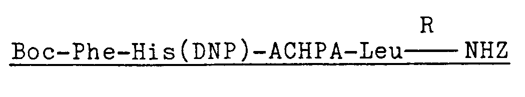 Zu 192 mg Boc-Phe-His(DNP)-OH, 30 µl Pyridin und 50 µl N-Ethylpiperidin in 10 ml CH₂Cl₂ wird bei - 5°C 44 µl Pivaloylchlorid zugegeben. 30 min wird bei +5 bis +10°C gerührt, auf -10°C gekühlt und 165 mg 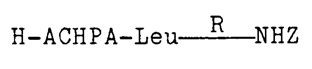 in 5 ml CH₂Cl₂ zugetropft. Man rührt 16 h ohne Kühlung, engt im Vakuum ein und nimmt in 100 ml EE auf. Man extrahiert 3 mal mit wäßriger K₂CO₃-Lösung, 1 mal mit H₂O, trocknet über Na₂SO₄ und engt im Vakuum ein. Nach Chromathographie an Kieselgel (Laufmittel EE/Methanol 10:1) erhält man die Titelverbindung als schwach gelbliches Pulver.
   R_{f} (EE/Methanol 10:1) = 0,5 ; MS(FAB) : 998 (M+1)
c) 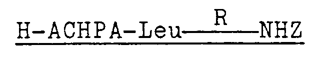 200 mg 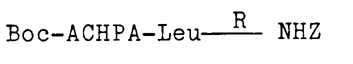 werden in 5 ml Dioxan gelöst. Unter leichter Kühlung (+ 10°C) werden 10 ml 12 %ige HCl in Dioxan zugegeben. Nach 3 h bei R.T. wird im Vakuum eingeengt, in H₂O aufgenommen, mit gesättigter wäßriger Na₂CO₃-Lösung auf pH = 9-10 eingestellt und 3 mal mit EE extrahiert. Nach Trocknung mit Na₂SO₄ und Einengen im Vakuum erhält man die Titelverbindung, die ohne weitere Reinigung in den nächsten Kupplungsschritten verwendet werden kann.
d) Boc-Phe-His(DNP)-OH wird nach der Aktivestermethode aus Boc-Phe-O-N-Suc und H-His(DNP)-OH hergestellt (J. Amer. Chem. Soc. 86 [1964] 1839).
e) 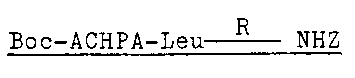 Zu 250 mg Boc-ACHPA-OH (Herstellung nach J. Med. Chem. 28 1985 , 1779) und 160 mg HOBt in 10ml THF werden bei 0°C 180 mg DCC gelöst in 5 ml THF zugetropft. Nach 1 h ohne Kühlung gibt man bei 0°C
   279 mg 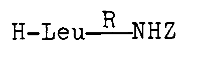 in 10 ml THF zu. Nach 16 h bei R.T. wird filtriert, eingeengt und in EE gelöst. Man extrahiert 2 mal mit gesättigter wäßriger Na₂CO₃-Lösung und einmal mit H₂O. Nach Trocknen mit Na₂SO₄ wird eingeengt. Nach Chromatographie an Kieselgel (Laufmittel EE/n-Hexan 2:1) erhält man die Titelverbindung als farbloses Pulver.
   R_{f} (EE/n-Hexan) = 0,7; MS (FAB) : 548 (M+1)
f) 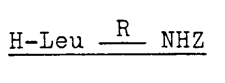 Analog zu Referenzbeispiel 1 c), ausgehend von 350 mg 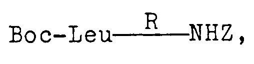 wird die Titelverbindung als Schaum erhalten, der ohne weitere Reinigung für die folgende Kupplung verwendet wird.
g) 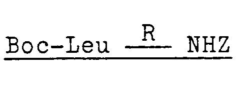 600 mg 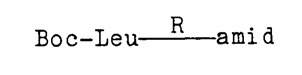 und 693 mg Z-0-Succinimid, gelöst in 10 ml DMF werden 16 h bei R.T. gerührt. Nach Einengen im Vakuum wird in EE gelöst, 2 mal mit gesättigter wäßriger NaCl-Lösung, 2 mal mit gesättigter wäßriger KHSO₄-Lösung und einmal mit gesättigter wäßriger NaCl-Lösung extrahiert. Nach Einengen im Vakuum erhält man die Titelverbindung als farbloses Öl, das ohne weitere Reinigung weiter verwendet wird.
   R_{f} (EE) = 0,8; MS (FAB) : 351 (M+1)
h) 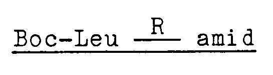 2,8 g Boc-Leu-nitril (Herstellung analog J.Amer. Chem. Soc. 88 (1966), 2033) werden mit vorbehandeltem Raney-Ni (Vorbehandlung: (1) gut mit H₂O gewaschen, (2) mit Decalin erhitzt und dekantiert, (3) in Methanol suspendiert und dekantiert) in 100 ml verdünntem ammoniakalischem Methanol 2 h bei R.T. hydriert. Nach Filtration und Einengen im Vakuum wird mit wäßriger 2N Zitronensäure auf pH=2 eingestellt und 3 mal mit CH₂Cl₂ extrahiert. Nach Einstellen der wäßrigen Phase auf pH=8-9 mit wäßriger 2N NaOH-Lösung wird abermals 3 mal mit CH₂Cl₂ extrahiert. Die vereinigten organischen Extrakte werden einmal mit H₂O gewaschen, mit Na₂SO₄ getrocknet und eingeengt. Das schwach gelbe Öl kann ohne weitere Reinigung für die weiteren Reaktionen verwendet werden.
   R_{f} (Methanol/1 % NH₃)=0,6; MS : 217 (M+1)

### Beispiel 1

### Iva-Phe-His-1(S)-cyclohexylmethyl-2(R,S)-hydroxy-3-(2-pyridyl)-propylamid

380 mg Iva-Phe-His(DNP)-1(S)-cyclohexylmethyl-2-(R,S)-hydroxy-3-(2-pyridyl)-propylamid werden analog Referenzbeispiel 1a) mit Thiophenol zur Titelverbindung umgesetzt.

R_{f} (EE/Methanol 5:1)=0,15 ; MS(FAB) : 617(M+1)

### a) Iva-Phe-His(DNP)-1(S)-cyclohexylmethyl-2(R,S)-hydroxy-3-(2-pyridyl)-propylamid

610 mg Iva-Phe-His(DNP)-OH und 300 mg 1(S)-Cyclohexylmethyl-2(R,S)-hydroxy-3-(2-pyridyl)propylamin werden analog Referenzbeispiel 1b) nach der Pivaloylchlorid-Methode zur Titelverbindung umgesetzt.

R_{f} (EE/Methanol 10:1)=0,3 ; MS(FAB) : 783(M+1)

### b) 1(S)-Cyclohexylmethyl-2(R,S)-hydroxy-3-(2-pyridyl)-propylamin

400 mg Boc-[1(S)-cyclohexylmethyl-2(R,S)-hydroxy-3-(2-pyridyl)]-propylamin werden mit Dioxan/HCl analog Referenzbeispiel 1c) zur Titelverbindung umgesetzt.

### c) Boc 1(S)-cyclohexylmethyl-2(R,S)-hydroxy-3-(2-pyridyl) - propylamin

Zu 2 ml 2-Methylpyridin, gelöst in 20 ml THF, werden bei -30°C 13 ml n-Butyllithium (15 % in Hexan) zugegeben. Man rührt 1 h ohne Kühlung tropft dann bei 10°C 2,55 g Boc-cyclohexylalaninal in 25 ml THF zu. Unmittelbar danach werden 10 ml H₂O zugegeben. Nun wird 3 mal mit EE extrahiert, mit Na₂SO₄ getrocknet und im Vakuum eingeengt. Chromatographie an Kieselgel (Laufmittel EE/n-Hexan 1:1) liefert die Titelverbindung als gelbliches Öl, wobei die Diasteromeren getrennt erhalten werden. (Dies trifft auch auf alle analogen Beispiele zu).
- R_{f} (EE/n-Hexan): =0,15 (polares Diastereomer)
=0,25 (weniger polares Diastereomer)

MS:349(M+1)

### Beispiel 2

### Iva-Phe-His-1(S)-cyclohexylmethyl-2(R,S)-hydroxy-3-(1-methylimidazol-2-yl)-propylamid:

328 mg Iva-Phe-His(DNP)-1(S)-cyclohexylmethyl-2(S)-hydroxy-3-(1-methylimidazol-2-yl)-propylamid werden analog Referenzbeispiel 1a) mit Thiophenol zur Titelverbindung umgesetzt.

R_{f} (Aceton/Wasser 10:1)=0,05 ; MS(FAB) : 620(M+1)

### a) Iva-Phe-His(DNP)-1(S)-cyclohexylmethyl-2(R,S)-hydroxy-3-(1-methylimidazol-2-yl)-propylamid:

Analog Referenzbeispiel 1b) werden 717 mg Iva-Phe-His(DNP)-OH und 325 mg 1(S)-Cyclohexylmethyl-2(S)-hydroxy-3-(1-Methylimidazol-2-yl)-propylamin mit Pivaloylchlorid gekuppelt.

R_{f} (Acetonitril/Wasser 8:1)=0,11; MS(FAB) : 786(M+1)

### b) 1(S)-Cyclohexylmethyl-2(R,S)-hydroxy-3-(1-methylimidazol-2-yl)-propylamin:

790 mg 4(S)-Cyclohexylmethyl-5-(1-methylimidazol-2-yl-methyl)-1,3-oxazolidin-2-on werden in 33 ml 2N LiOH in MeOH/H₂O 1:1 gelöst und 3 h unter Rückfluß erhitzt, anschließend mit 150 ml Wasser verdünnt und 3 mal mit 50 ml CH₂Cl₂ extrahiert. Nach der Trocknung mit Na₂SO₄ und Einengung im Vakuum erhält man die Titelverbindung als farbloses Öl, das ohne Reinigung weiter eingesetzt wird.

R_{f} (EE/MeOH)=0,11

### c) 4(S)-Cyclohexylmethyl-5-(R,S)-(1-methylimidazol-2-yl) methyl-1,3-oxazolidin-2-on

Zu 3,23 g 1,2-Dimethylimidazol gelöst in 70 ml THF werden bei -40°C 21,6 ml 1,6 M n-Butyllithium-Lösung in Hexan zugetropft. Nach 40 min. Rühren bei 0°C wird bei -30°C 4,31 g Z-Cyclohexylalaninal (hergestellt nach Synthesis 1983, 676) gelöst in 35 ml THF zugetropft. Man läßt auf R.T. erwärmen und rührt das Reaktionsgemisch in 100 ml gesättigter wäßriger NaHCO₃-Lösung ein. Das THF wird im Vakuum entfernt und die wäßrige Phase 3 mal mit 100 ml EE extrahiert. Nach Trocknen mit Na₂SO₄ und Einengen im Vakuum wird an Kieselgel chromatographiert (MTB/MeOH 9:1) und man erhält die Titelverbindung als farbloses Öl.

R_{f} (MTB/MeOH 8:1)=0,17 ; MS(FAB) : 278(M+1)
Die bei 270 MHZ gemessenen ¹H-NMR-Spektren erwiesen sich bei allen Titelverbindungen als in Einklang mit den angegebenen Strukturen.

### Beispiel 3

### Etoc-Phe-His-1(S)-Cyclohexylmethyl-2(R,S)-hydroxy-3-(4,4-dimethyloxazolin-2-yl)propylamid

300 mg Etoc-Phe-His(DNP)-1(S)-Cyclohexylmethyl-2-hydroxy-3-(4,4-dimethyloxazolin-2-yl)propylamid werden analog Referenzbeispiel 1a) mit Thiophenol zur Titelverbindung umgesetzt.

R_{f} (Acetonitril/H₂O 10:1) = 0,2; MS (FAB) : 625 (M+1)
a) Etoc-Phe-His(DNP)-1(S)-Cyclohexylmethyl-2(R,S)-hydroxy-3-(4,4-dimethyloxazolin-2-yl)propylamid
   490 mg Boc-His(DNP)-1(S)-Cyclohexylmethyl-2-hydroxy-3-(4,4-dimethyloxazolin-2-yl)propylamid werden mit 169 mg Etoc-Phe analog Beispiel 1b) umgesetzt.
   R_{f} (EE/MeOH 10:1) = 0,4; MS(FAB) : 789 (M+1)
b) Boc-His(DNP)-1(S)-Cyclohexylmethyl-2(R,S)-hyrdroxy-3-(4,4-dimethyloxazolin-2-yl)propylamid
   260 mg Boc-1(S)-Cyclohexylmethyl-2-hydroxy-3-(4,4-dimethyloxazolin-2-yl)propylamid werden mit 299 mg Boc-His(DNP)-OH analog Beispiel 1c) und 1d) umgesetzt.
   R_{f} (EE) = 0,3; MS (FAB) : 670 (M+1)
c) Boc-1(S)-Cyclohexylmethyl-2(R,S)-hydroxy-3-(4,4-dimethyloxazolin-2-yl)propylamid
   1,4 g Boc-Cyclohexylalaninal werden nach Meyers, J. Org. Chem. 39, 2778 (1974) mit 1,4 ml 2,4,4-Trimethyloxazolin umgesetzt. Man erhält 2 Diastereomere im Verhältnis 1:1 D₁ : R_{f} (MTB/Diisopropylether 1:1) = 0,15; MS : 369 (M+1) D₂: R_{f} (MTB/Diisopropylether 1:1) = 0,10; MS : 369 (M+1) Zur Herstellung der Titelverbindung in Beispiel 3 wird D₁ weiterverarbeitet.

Die Verbindungen der Beispiele 4 und 5 werden analog Beispiel 3 hergestellt.

### Beispiel 4

### Etoc-Phe-His-1(S)-Cyclohexylmethyl-2(R,S)-hydroxy-3-(4(S,S)-sek.-butyl-oxazolin-2-yl)propylamid

R_{f} (Acetonitril/H₂O 10:1) = 0,3; MS (FAB) : 653 (M+1)

### Beispiel 5

### Iva-Thi-His-1(S)-Cyclohexylmethyl-2(R,S)-hydroxy-3-(4(S,S)-sek.-butyl-oxazolin-2-yl)propylamid

R_{f} (Acetonitril/H₂O 10:1) = 0,2; MS (FAB) : 671 (M+1)
Das in Beispiel 4 und 5 verwendete Oxazolin wurde wie folgt erhalten:
a) 4(S,S)-sek.-Butyl-2-methyl-oxazolin
   15 g 2-Acetylamino-3-methyl-pentanol werden in 200 ml CH₂Cl₂ gelöst und bei 0 °C unter Argon 3,5 ml Thionylchlorid zugetropft und 3 h bei 0 °C gerührt. Die Reaktionslösung wird auf 200 ml gesättigte wäßrige Na₂CO₃-Lösung gegossen und 3 mal mit 100 ml EE extrahiert. Nach Trocknung mit Na₂SO₄ und Einengen im Vakuum erhält man neben der Titelverbindung noch Bis-(2-Acetylamino-3-methyl-pentyl)sulfit, das durch Erhitzen auf 110 °C in Toluol ebenfalls noch in die Titelverbindung umgewandelt werden kann.
   R_{f} (MTB/Aceton 10:1) = 0,3; MS : 142 (M+1)
b) 2-Acetylamino-3-methyl-pentanol
   22,4 g Ac-Ile-OMe werden in 200 ml THF gelöst, bei 0 °C mit 8,9 g LiAlH₄ versetzt und 1 h bei 0 °C gerührt. Etwa 90 % des THF werden im Vakuum entfernt, mit 100 ml 5 %iger wäßriger KHSO₄-Lösung versetzt und 3 mal mit 200 ml EE extrahiert. Nach Trocknung über Na₂SO₄ und Einengen im Vakuum wird an Kieselgel chromatographiert (Laufmittel MTB/Aceton 10:1) und man erhält die Titelverbindung als farbloses Öl.
   R_{f} (MTB/Aceton 10:1) = 0,1; MS : 160 (M+1)

### Beispiel 6

### Iva-Thi-His-1(S)-Cyclohexylmethyl-2(R,S)-hydroxy-4-(4(S,S)-sek.-butyl-oxazolin-2-yl)butylamid

Analog Referenzbeispiel 1a) und 1b) werden 260 mg 1(S)-Cyclohexylmethyl-2(S)-hydroxy-4-(4(S,S)-sek.-butyl-oxazolin-2-yl)butylamin mit dem N-terminalen Dipeptid verknüpft.

R_{f} (Acetonitril/H₂O 10:1) = 0,2; MS (FAB) : 685 (M+1)
a) 1(S)-Cyclohexylmethyl-2(R,S)-hydroxy-4-(4(S,S)-sek.-butyl-oxazolin-2-yl)butylamin
   Analog Referenzbeispiel 1c) werden 570 mg 3-Boc-4(S)-cyclohexylmethyl-2,2-dimethyl-5(S)- 2-(4(S,S)-sek.-butyl-oxazolin-2-yl)-ethyl oxazolidin entschützt und ohne Reinigung weiter umgesetzt.
b) 3-Boc-4(S)-cyclohexylmethyl-2,2-dimethyl-5(R,S)-[2-(4(S,S)-sek.-butyl-oxazolin-2-yl)-ethyl]oxazolidin
   3,76 g 4(S,S)-sek.-Butyl-2-methyl-oxazolin und 4,0 ml N,N,N',N'-Tetramethylethylendiamin werden in 100 ml THF gelöst und bei -78 °C 14,9 ml einer 1,6 M Lösung von n-Butyllithium in Hexan zugetropft. 30 min. wird bei dieser Temperatur gerührt, anschließend 1,15 g 3-Boc-4(S)-cyclohexylmethyl-2,2-dimethyl-5(R,S)-iodmethyl-oxazolidin in 10 ml THF zugetropft. 1 h wird bei R.T. gerührt, die Reaktionslösung in 200 ml gesättigte wäßrige NaHCO₃-Lösung gegossen und 3 mal mit 200 ml EE extrahiert. Nach Trocknung über Na₂SO₄ und Einengen im Vakuum wird an Kieselgel chromatographiert (Laufmittel MTB/Diisopropylether 1:1). Man erhält die Titelverbindung als farbloses Öl.
   R_{f} (MTB/Diisopropylether 1:1) = 0,6; MS (FAB) : 451 (M+1)
c) 3-Boc-4(S)-cyclohexylmethyl-2,2-dimethyl-5(R,S)-iodmethyl-oxazolidin
   2,4 g Boc-2-cyclohexyl-1(S)-oxiran-2(R,S)-yl-ethylamin, 1,3 g NaI und 1,1 ml Trimethylsilylchlorid werden in 100 ml Acetonitril gelöst und 1,5 h bei R.T. gerührt. Anschließend werden 8,7 ml einer 1 M Lösung von Tetrabutylammoniumfluorid in THF und 5,3 ml Dimethyoxyethan zugespritzt. 2,5 h wird bei R.T. gerührt, anschließend mit 200 ml gesättigter wäßriger NaHCO₃-Lösung versetzt und 3 mal mit 200 ml MTB extrahiert. Nach Trocknung über Na₂SO₄ und Einengen im Vakuum wird an Kieselgel chromatographiert (Laufmittel MTB/Diisopropylether 1:2) und man erhält die Titelverbindung als farbloses Öl.
   R_{f} (MTB/Diisopropylether 1:2) = 0,5; MS : 422 (M-CH₃)
d) Boc-2-cyclohexyl-1(S)-oxiran-2(R,S)-yl-ethylamin
   2,6 g Boc-1(S)-cyclohexylmethyl-prop-2-en-1-yl-amin werden in 50 ml CH₂Cl₂ gelöst und 1,9 g m-Chlorperbenzoesäure in 50 ml CH₂Cl₂ bei 0 °C zugetropft. 36 h wird bei R.T. gerührt, anschließend die Reaktionslösung zunächst mit 100 ml 5 %iger wäßriger Na₂SO₃-Lösung, dann mit 100 ml gesättigter wäßriger Na₂CO₃-Lösung extrahiert. Nach Trocknung über Na₂SO₄ und Einengen im Vakuum wird an Kieselgel chromatographiert (Laufmittel MTB/Cyclohexan 1:1) und man erhält die Titelverbindung als farbloses Öl, Diastereomerenverhältnis 3:1 bis 5:1
   Diastereomerengemisch:
   R_{f} (MTB/Cyclohexan 1:1) = 0,4; MS (FAB) : 270 (M+1)
e) Boc-1(S)-cyclohexylmethyl-prop-2-en-1-yl-amin
   5,4 g Methyl-triphenylphosphoniumbromid werden in 100 ml THF suspendiert und bei R.T. 1,7 g Kalium-t-butylat unter Argon zugegeben. 2 h wird bei R.T. gerührt, auf 0 °C abgekühlt und 3,9 g Boc-Cyclohexylalaninal in 50 ml THF zugetropft. 30 min. wird bei 0 °C nachgerührt, 100 ml H₂O zugetropft und das THF im Vakuum entfernt. 100 ml gesättigte wäßrige NaHCO₃-Lösung werden addiert und 3 mal mit 100 ml CH₂Cl₂ extrahiert. Nach Trocknung über Na₂SO₄ und Einengen im Vakuum wird an Kieselgel chromatographiert (Laufmittel MTB/Cyclohexan 1:3) und man erhält die Titelverbindung als farbloses Öl.
   R_{f} (MTB/Cyclohexan 1:3) = 0,4; MS : 254 (M+1)

Die Verbindungen der Beispiele 7-9 werden analog Beispiel 1 hergestellt.

### Beispiel 7

### Iva-Phe-His-1(S)-cyclohexylmethyl-2(R,S)-hydroxy-3-(2-benzoxazolyl)-propylamid

R_{f} (EE/MeOH 5:1) = 0,3; MS (FAB) = 657 (M+1)

### Beispiel 8

### Iva-Phe-His-1(S)-cyclohexylmethyl-2-hydroxy-3-(4-pyridyl)-propylamid 1. Diastereomer

R_{f} (EE/MeOH 3:1) = 0,2; MS(FAB) = 617 (M+1)

### Beispiel 9

### Iva-Phe-His-1-(S)-cyclohexylmethyl-2-hydroxy-3-(4-pyridyl)-propylamid 2. Diastereomer

R_{f} (EE/MeOH 5:1) = 0,1; MS (FAB) = 617 (M+1)

### Beispiel 10

### Iva-Phe-His-1(S)-cyclohexylmethyl-2(R,S)-hydroxy-4-(2-pyridyl)-butylamid

Die Titelverbindung wird analog Referenzbeispiel 1a) durch Umsetzung von 400 ml Iva-Phe-His(DNP)-1(S)-cyclohexylmethyl-2(R,S)-hydroxy-4-(2-pyridyl)-butylamid mit Thiophenol synthetisiert.

R_{f} (EE/MeOH 5:1) = 0,15; MS (FAB) = 631 (M+1)
a) Iva-Phe-His(DNP)-1(S)-cyclohexylmethyl-2(R,S)-hydroxy-4-(2-pyridyl)-butylamid
   Die Titelverbindung wird analog Referenzbeispiel 1b) und 1c) aus 552 mg Iva-Phe-His(DNP)-OH und 400 mg Boc-1(S)-cyclohexylmethyl-2-(R,S)-hydroxy-4-(2-pyridyl)-butylamin hergestellt.
   R_{f} (EE/MeOH 10:1) = 0,25; MS (FAB) 797 (M+1)
b) Boc-1(S)-cyclohexylmethyl-2(R,S)-hydroxy-4-(2-pyridyl)-butylamin
   220 µl 2-Picolin (2 eq) werden analog Beispiel 1c deprotoniert. Anschließend werden 300 mg Boc-2-cyclohexyl-1(S)-oxiran-2(R,S)-yl-ethylamin in THF bei -60 °C zugetropft. Nach 1 h wird H₂O zugegeben und 3 mal mit EE extrahiert. Nach dem Trocknen der organischen Phasen mit Na₂SO₄ wird eingeengt und die Titelverbindung durch Chromatographie an SiO₂ mit EE/H 2:1 als Eluens isoliert.
   R_{f} (EE/H 2:1) = 0,3; MS (FAB) = 363 (M+1)

### Beispiel 11

### Iva-Phe-His-1(S)-cyclohexylmethyl-2(S)-hydroxy-5-(2-pyridyl)-pentylamid

Die Titelverbindung wird analog Referenzbeispiel 1a) durch Behandeln von 110 mg Iva-Phe-His(DNP)-1(S)-cyclohexylmethyl-2(S)-hydroxy-5-(2-pyridyl)-pentylamid mit Thiophenol hergestellt.

R_{f} (Acetonitril/H₂O 8:1) = 0,3; MS (FAB) = 645 (M+1)
a) Iva-Phe-His(DNP)-1(S)-cyclohexylmethyl-2(S)-hydroxy-5-(2-pyridyl)-pentylamid
   Die Titelverbindung wird analog Referenzbeispiel 1b) und 1c) aus 263 mg Iva-Phe-His(DNP)-OH und 200 mg 3-Boc-4(S)-cyclohexylmethyl-2,2-dimethyl-5-(3-(2-pyridyl)-propyl)-oxazolidin synthetisiert.
   R_{f} (EE/MeOH) = 0,4; MS (FAB) = 811 (M+1)
b) 3-Boc-4(S)-cyclohexylmethyl-2,2-dimethyl-5-(3-(2-pyridyl)-propyl)-oxazolidin
   367 µl 2-Picolin wird analog Beispiel 1c deprotoniert. Zur tiefroten Lösung wird bei -50 °C 500 mg 3-Boc-4(S)cyclohexylmethyl-2,2-dimethyl-5-(2-bromethyl)-oxazolidin in 10 ml THF zugegeben. Nach 15 min. wird H₂O addiert und 3 mal mit EE extrahiert. Nach dem Trocknen der organischen Phasen mit Na₂SO₄ wird eingeengt und an Kieselgel chromatographiert (Eluens: Toluol/EE 3:1)
   R_{f} (Toluol/EE 3:1) = 0,25; MS = 417 (M+1)
c) 3-Boc-1(S)-cyclohexylmethyl-2,2-dimethyl-5-(2-bromethyl)-oxazolidin
   Zu 690 mg 3-Boc-1(S)-cyclohexylmethyl-2,2-dimethyl-5-(2-hydroxyethyl)-oxazolidin, 2,6 g Triphenylphosphin und 1,6 g Pyridiniumbromid in 15 ml CH₂Cl₂ werden unter Argon 1,6 ml Azodicarbonsäurediethylester bei 20 °C zugetropft. Nach 16 h bei R.T. wird H₂O zugegeben und mit 100 ml CH₂Cl₂ verdünnt. Die organische Phase wird 2 mal mit gesättigter NaHCO₃-Lösung und 1 mal mit gesättigter NaCl-Lösung gewaschen. Die mit Na₂SO₄ getrocknete organische Phase wird eingeengt, der Rückstand in wenig EE aufgenommen und zur Abtrennung von PPh₃ filtriert. Reinigung an Kieselgel liefert die Titelverbindung (Eluens: H/EE 15:1).
   R_{f} (H/EE 15:1) = 0,3; MS 404 (M)
d) 3-Boc-1(S)-cyclohexylmethyl-2,2-dimethyl-5-(2-hydroxymethyl)-oxazolidin
   10 g Boc-ACHPA-OEt, 500 mg p-Toluolsulfonsäure und 7,2 ml Dimethoxypropan werden in 160 ml Toluol unter Argon 2 h auf 80 °C erhitzt. Anschließend wird eingeengt. Der Rückstand wird bei 0 °C zu einer Suspension aus 2 g LiAlH₄ in 200 ml THF zugetropft. Nach 2,5 h bei 0 °C werden 100 ml 5 %ige NaHSO₄-Lösung zugegeben und 3 mal mit EE extrahiert. Die vereinigten organischen Phasen werden 1 mal mit gesättigter NaHCO₃-Lösung gewaschen. Nach dem Trocknen mit Na₂SO₄ wird eingeengt und Chromatographiert (Eluens: H/EE 2:1).
   R_{f} (H/EE 4:1) = 0,1; MS = 342 (M+1)

Beispiele 12-15 werden analog Beispiel 11 synthetisiert.

### Beispiel 12

### Iva-Phe-His-1(S)-cyclohexylmethyl-2(S)-hydroxy-5-(4-pyridyl)-pentylamid

R_{f} (Acetonitril/H₂O 10:1) = 0,1; MS = 644 (M)

### Beispiel 13

### Iva-Phe-His-1(S)-cyclohexylmethyl-2(S)-hydroxy-5-(4(S,S)-sek.-butyl-oxazolin-2-yl)-pentylamid

R_{f} (Acetonitril/H₂O 10:1) = 0,1; MS (FAB) = 693 (M+1)

### Beispiel 14

### Iva-Phe-His-1(S)-cyclohexylmethyl-2(S)-hydroxy-5-(2-ethyl-4-pyridyl)-pentylamid

R_{f} (CH₂Cl₂/MeOH/CH₃COOH/H₂O 80:20:1:1) = 0,5; MS (FAB) : 673 (M+1)

### Beispiel 15

### Iva-Phe-His-1(S)-cyclohexylmethyl-2(S)-hydroxy-5-(4-ethyl-2-pyridyl)-pentylamid

R_{f} (Acetonitril/H₂O 10:1) = 0,3; MS (FAB) : 673 (M+1)

### Beispiel 16

### Iva-Phe-His-1(S)-cyclohexylmethyl-2(S)-hydroxy-5-(4-piperidinyl)-pentylamid

Die Titelverbindung wird aus Boc-1(S)-cyclohexylmethyl-2(S)-hydroxy-5(4-benzyloxycarbonylpiperidinyl)-pentylamid analog Referenzbeispiel 1, 1a) - 1c) hergestellt.

R_{f} (Acetonitril/H₂O 2:1) = 0,3; MS (FAB) = 651 (M+1)
a) Boc-1(S)-cyclohexylmethyl-2(S)-hydroxy-5(4-benzyloxycarbonylpiperidinyl)-pentylamid
   265 mg 3-Boc-4(S)-cyclohexylmethyl-2,2-dimethyl-5-(3-(4-pyridyl)-propyl)-oxazolidin werden über 40 mg 5 % Rhodium auf Kohle in 50 ml Ethanol 3,5 h bei 100 °C und 100 bar hydriert. Anschließend wird vom Katalysator abfiltriert und eingeengt. Der Rückstand wird in 5 ml DMF gelöst und nach Zugabe von 160 mg Z-0-Succinimid 16 h bei R.T. gerührt. Anschließend wird eingeengt und an Kieselgel chromatographiert (Eluens: H/EE 4:1).
   R_{f} (H/EE) = 0,4; MS = 541 (M-CH₃)

Die Verbindungen der Beispiele 17 und 18 werden analog Beispiel 11 hergestellt.

### Beispiel 17

### Etoc-Phe-His-1(S)-Cyclohexylmethyl-2(S)-hydroxy-5-(4,4-dimethyloxazolin-2-yl)-pentylamid

R_{f} (Acetonitril/H₂O 10:1) = 0,1; MS (FAB) : 653 (M+1)

### Beispiel 18

### Iva-Phe-His-1(S)-Cyclohexylmethyl-2(S)-hydroxy-5-(4,5-dimethyloxazol-2-yl)-pentylamid

R_{f} (EE/MeOH 5:1) = 0,2; MS (FAB) : 663 (M+1)

## Patentansprüche (Patentansprüche für folgende(n) Vertragsstaat(en): BE, CH, DE, FR, GB, IT, LI, LU, NL, SE)

1. Verbindung der Formel I 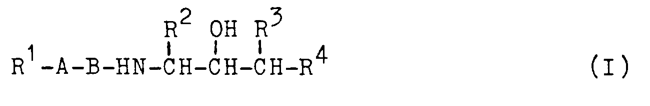 in welcher
R¹ einen Rest der Formel II bedeutet,
R^{a}-W- (II)
worin W für -CO-, -O-CO-, -SO₂- oder -NH-CO-steht und R^{a} Wasserstoff, (C₁-C₁₀)-Alkyl, das gegebenenfalls ein- oder zweifach ungesättigt ist und das gegebenenfalls durch bis zu 3 gleiche oder verschiedene Reste aus der Reihe Hydroxy, (C₁-C₇)-Alkoxy, (C₁-C₇)-Alkanoyloxy, Carboxy, (C₁-C₇)-Alkoxycarbonyl, Cl, Br, Amino, (C₁-C₇)-Alkylamino, Di-(C₁-C₇)-alkylamino, (C₁-C₅)-Alkoxycarbonylamino, (C₇-C₁₅)-Aralkoxycarbonylamino und 9-Fluorenylmethyloxycarbonylamino substituiert ist, (C₃-C₈)-Cycloalkyl, (C₃-C₈)-Cycloalkyl-(C₁-C₆)-alkyl, (C₆-C₁₄)-Aryl, das gegebenenfalls durch einen oder zwei gleiche oder verschiedene Reste aus der Reihe F, Cl, Br, J, Hydroxy, (C₁-C₇)-Alkoxy, (C₁-C₇)-Alkyl, (C₁-C₇)-Alkoxycarbonyl, Amino, gegebenenfalls mit bis zu 2 Halogen substituiertes Anilino und Trifluormethyl substituiert ist, (C₆-C₁₄)-Aryl-(C₁-C₆)-alkyl, worin der Arylteil gegebenenfalls durch einen oder zwei gleiche oder verschiedene Reste aus der Reihe F, Cl, Br, J, Hydroxy, (C₁-C₇)-Alkoxy, (C₁-C₇)-Alkyl, (C₁-C₇)-Alkoxycarbonyl, Amino, (C₁-C₇)-Alkylamino, Di-(C₁-C₇)-alkylamino, Carboxy, Carboxymethoxy, Amino-(C₁-C₇)-alkyl, (C₁-C₇)-Alkylamino-(C₁-C₇)-alkyl, Di-(C₁-C₇)-alkylamino-(C₁-C₇)-alkyl, (C₁-C₇)-Alkoxycarbonylmethoxy, Carbamyl, Sulfamoyl, (C₁-C₇)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom als Ringglieder steht, der gegebenenfalls durch einen oder zwei gleiche oder verschiedene Reste aus der Reihe F, Cl, Br, Hydroxy, (C₁-C₇)-Alkoxy, (C₁-C₇)-Alkyl, (C₁-C₇)-Alkoxycarbonyl, Amino oder Trifluormethyl substituiert ist,
R⁴ einen Rest der Formel IV bedeutet,
- X - (CH₂)_{n'} - R⁷ (IV)
A einen N-terminal mit R¹ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, Norvalin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methylcystein, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,
B einen Rest einer Aminosäure, wie unter A definiert, bedeutet,
R² Wasserstoff, (C₁-C₁₀)-Alkyl, (C₄-C₇)-Cycloalkyl, (C₄-C₇)-Cycloalkyl-(C₁-C₄)-alkyl, (C₆-C₁₄)-Aryl oder (C₆-C₁₄)-Aryl-(C₁-C₄)-alkyl bedeutet und
R³ Wasserstoff, (C₁-C₁₀)-Alkyl, (C₆-C₁₄)-Aryl oder (C₆-C₁₄)-Aryl-(C₁-C₄)-alkyl bedeutet,
X abwesend ist,
n' = 0, 1, 2, 3 oder 4 sein kann,
R⁷ Heteroaryl, das auch teilweise hydriert sein kann
sowie deren physiologisch verträglichen Salze, wobei Verbindungen ausgenommen sind, bei denen R²=butyl, R³=H und R⁷=4-imidazol ist.

2. Verbindung der Formel I gemäß Anspruch 1, in welcher R¹ (C₁-C₁₁)-Alkanoyl, vorzugsweise n-Decanoyl, Formyl, Acetyl, Pivaloyl, Isovaleryl oder Isobutyryl, gegebenenfalls geschütztes Amino-(C₁-C₁₁)-alkanoyl, vorzugsweise 4-Aminobutyryl, 5-Aminopentanoyl, 6-Aminohexanoyl, 4-N-tert.-Butoxycarbonylaminobutyryl, 5-N-tert.Butoxycarbonylaminopentanoyl oder 6-N-tert.-butoxycarbonylaminohexanoyl, Di-(C₁-C₇)-alkylamino-(C₂-C₁₁)-alkanoyl, vorzugsweise Dimethylaminoacetyl, (C₄-C₉)-Cycloalkylcarbonyl, vorzugsweise Cyclopropylcarbonyl, Cyclobutylcarbonyl, Cyclopentylcarbonyl oder Cyclohexylcarbonyl, (C₆-C₁₀)-Aryl-(C₂-C₁₁)-alkanoyl, vorzugsweise Phenylacetyl, Phenylpropanoyl oder Phenylbutanoyl, 2-(2,6-Dichloroanilino)-phenylacetyl, 2-(N-Benzyl-2,6-dichloranilino)-phenylacetyl, gegebenenfalls durch Halogen, (C₁-C₇)-Alkyl, (C₁-C₇)-Alkoxy, (C₁-C₇)-Alkoxycarbonyl substituiertes Benzoyl, vorzugsweise 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzolsulfonyl, (C₁-C₁₀)-Alkoxycarbonyl, vorzugsweise Methoxycarbonyl, Ethoxycarbonyl oder tert.-Butoxy-carbonyl, durch Halogen substituiertes (C₁-C₁₀)-Alkoxycarbonyl, vorzugsweise 2,2,2-Trichlorethoxycarbonyl oder 1,1-Dimethyl-2,2,2-trichlorethoxycarbonyl, (C₆-C₁₄)-Aryl-(C₁-C₆)-alkoxycarbonyl, vorzugsweise Benzyloxycarbonyl oder 9-Fluorenylmethylcarbonyl, bedeutet sowie deren physiologisch verträglichen Salze.

3. Verbindung der Formel I gemäß einem oder mehreren der Ansprüche 1-2, in welcher
R² Isobutyl, Benzyl oder Cyclohexylmethyl bedeutet, sowie deren physiologisch verträglichen Salze.

4. Verbindung der Formel I gemäß einem oder mehreren der Ansprüche 1-3, in welcher
R³ Wasserstoff, Isopropyl oder Isobutyl bedeutet, sowie deren physiologisch verträglichen Salze.

5. Verfahren zur Herstellung einer Verbindung der Formel I gemäß einem der Ansprüche 1 bis 4, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

6. Pharmazeutisches Mittel enthaltend eine Verbindung gemäß einem der Ansprüche 1 bis 4.

## Patentansprüche (Patentansprüche für folgende(n) Vertragsstaat(en): AT, ES, GR)

1. Verfahren zur Herstellung einer Verbindung der Formel I 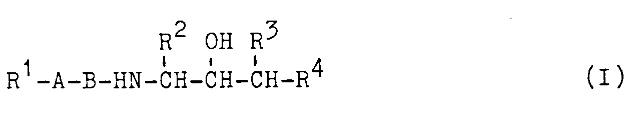 in welcher
R¹ einen Rest der Formel II bedeutet,
R^{a}-W- (II)
worin W für -CO-, -O-CO-, -SO₂- oder -NH-CO-steht und R^{a} Wasserstoff, (C₁-C₁₀)-Alkyl, das gegebenenfalls ein- oder zweifach ungesättigt ist und das gegebenenfalls durch bis zu 3 gleiche oder verschiedene Reste aus der Reihe Hydroxy, (C₁-C₇)-Alkoxy, (C₁-C₇)-Alkanoyloxy, Carboxy, (C₁-C₇)-Alkoxycarbonyl, Cl, Br, Amino, (C₁-C₇)-Alkylamino, Di-(C₁-C₇)-alkylamino, (C₁-C₅)-Alkoxycarbonylamino, (C₇-C₁₅)-Aralkoxycarbonylamino und 9-Fluorenylmethyloxycarbonylamino substituiert ist, (C₃-C₈)-Cycloalkyl, (C₃-C₈)-Cycloalkyl-(C₁-C₆)-alkyl, (C₆-C₁₄)-Aryl, das gegebenenfalls durch einen oder zwei gleiche oder verschiedene Reste aus der Reihe F, Cl, Br, J, Hydroxy, (C₁-C₇)-Alkoxy, (C₁-C₇)-Alkyl, (C₁-C₇)-Alkoxycarbonyl, Amino, gegebenenfalls mit bis zu 2 Halogen substituiertes Anilino und Trifluormethyl substituiert ist, (C₆-C₁₄)-Aryl-(C₁-C₆)-alkyl, worin der Arylteil gegebenenfalls durch einen oder zwei gleiche oder verschiedene Reste aus der Reihe F, Cl, Br, J, Hydroxy, (C₁-C₇)-Alkoxy, (C₁-C₇)-Alkyl, (C₁-C₇)-Alkoxycarbonyl, Amino, (C₁-C₇)-Alkylamino, Di-(C₁-C₇)-alkylamino, Carboxy, Carboxymethoxy, Amino-(C₁-C₇)-alkyl, (C₁-C₇)-Alkylamino-(C₁-C₇)-alkyl, Di-(C₁-C₇)-alkylamino-(C₁-C₇)-alkyl, (C₁-C₇)-Alkoxycarbonylmethoxy, Carbamyl, Sulfamoyl, (C₁-C₇)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom als Ringglieder steht, der gegebenenfalls durch einen oder zwei gleiche oder verschiedene Reste aus der Reihe F, Cl, Br, Hydroxy, (C₁-C₇)-Alkoxy, (C₁-C₇)-Alkyl, (C₁-C₇)-Alkoxycarbonyl, Amino oder Trifluormethyl substituiert ist,
R⁴ einen Rest der Formel IV bedeutet,
- X - (CH₂)_{n'} - R⁷ (IV)
A einen N-terminal mit R¹ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, Norvalin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, β-2-Benzo[b]thienylalanin, β-3-Benzo[b]thienylalanin, 2-Fluorphenylalanin, 3-Fluorphenylalanin, 4-Fluorphenylalanin, Norleucin, 1,2,3,4-Tetrahydroisochinolin-3-carbonsäure, Homophenylalanin, DOPA, O-Dimethyldopa, 2-Amino-4-(2-thienyl)-buttersäure, 2-Amino-4-(3-thienyl)-buttersäure, 3-(2-Thienyl)-serin, (Z)-Dehydrophenylalanin und (E)-Dehydrophenylalanin bedeutet,
B einen Rest einer Aminosäure, wie unter A definiert, bedeutet,
R² Wasserstoff, (C₁-C₁₀)-Alkyl, (C₄-C₇)-Cycloalkyl, (C₄-C₇)-Cycloalkyl-(C₁-C₄)-alkyl, (C₆-C₁₄)-Aryl oder (C₆-C₁₄)-Aryl-(C₁-C₄)-alkyl bedeutet und
R³ Wasserstoff, (C₁-C₁₀)-Alkyl, (C₆-C₁₄)-Aryl oder (C₆-C₁₄)-Aryl-(C₁-C₄)-alkyl bedeutet,
X abwesend
n' = 0, 1, 2, 3 oder 4 sein kann,
R₇ oder Heteroaryl, das auch teilweise hydriert sein kann bedeutet,
wobei Verbindungen ausgenommen sind, bei denen R²=butyl, R³=H und R⁷=4-imidazol ist,
sowie deren physiologisch verträglichen Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren zur Herstellung einer Verbindung der Formel I gemäß Anspruch 1, in welcher R¹ (C₁-C₁₁)-Alkanoyl, vorzugsweise n-Decanoyl, Formyl, Acetyl, Pivaloyl, Isovaleryl oder Isobutyryl, gegebenenfalls geschütztes Amino-(C₁-C₁₁)-alkanoyl, vorzugsweise 4-Aminobutyryl, 5-Aminopentanoyl, 6-Aminohexanoyl, 4-N-tert.-Butoxycarbonylaminobutyryl, 5-N-tert.Butoxycarbonylaminopentanoyl oder 6-N-tert.-butoxycarbonylaminohexanoyl, Di-(C₁-C₇)-alkylamino-(C₂-C₁₁)-alkanoyl, vorzugsweise Dimethylaminoacetyl, (C₄-C₉)-Cycloalkylcarbonyl, vorzugsweise Cyclopropylcarbonyl, Cyclobutylcarbonyl, Cyclopentylcarbonyl oder Cyclohexylcarbonyl, (C₆-C₁₀)-Aryl-(C₂-C₁₁)-alkanoyl, vorzugsweise Phenylacetyl, Phenylpropanoyl oder Phenylbutanoyl, 2-(2,6-Dichloroanilino)-phenylacetyl, 2-(N-Benzyl-2,6-dichloranilino)-phenylacetyl, gegebenenfalls durch Halogen, (C₁-C₇)-Alkyl, (C₁-C₇)-Alkoxy, (C₁-C₇)-Alkoxycarbonyl substituiertes Benzoyl, vorzugsweise 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzolsulfonyl, (C₁-C₁₀)-Alkoxycarbonyl, vorzugsweise Methoxycarbonyl, Ethoxycarbonyl oder tert.-Butoxy-carbonyl, durch Halogen substituiertes (C₁-C₁₀)-Alkoxycarbonyl, vorzugsweise 2,2,2-Trichlorethoxycarbonyl oder 1,1-Dimethyl-2,2,2-trichlorethoxycarbonyl, (C₆-C₁₄)-Aryl-(C₁-C₆)-alkoxycarbonyl, vorzugsweise Benzyloxycarbonyl oder 9-Fluorenylmethylcarbonyl bedeutet sowie deren physiologisch verträglichen Salze.

3. Verfahren zur Herstellung einer Verbindung der Formel I gemäß einem oder mehreren der Ansprüche 1-2, in welcher
R² Isobutyl, Benzyl oder Cyclohexylmethyl bedeutet, sowie deren physiologisch verträglichen Salze.

4. Verfahren zur Herstellung einer Verbindung der Formel I gemäß einem oder mehreren der Ansprüche 1-3, in welcher
R³ Wasserstoff, Isopropyl oder Isobutyl bedeutet, sowie deren physiologisch verträglichen Salze.

5. Verfahren zur Herstellung eines pharmazeutischen Mittels, enthaltend eine Verbindung der Formel I gemäß einem der Ansprüche 1-4, dadurch gekennzeichnet, daß man diese und einen Träger in eine geeignete Darreichungsform bringt.

## Claims (Claims for the following Contracting State(s): BE, CH, DE, FR, GB, IT, LI, LU, NL, SE)

1. A compound of the formula I 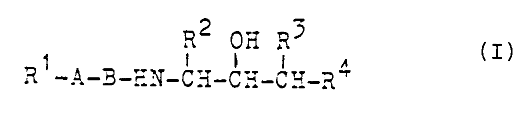 in which
R¹ denotes a radical of the formula II
R^{a}-W- (II)
in which W represents -CO-, -O-CO-, -SO₂- or -NH-CO-, and R^{a} represents hydrogen, (C₁-C₁₀)-alkyl which is optionally singly or doubly unsaturated and which is optionally substituted by up to 3 identical or different radicals from the series comprising hydroxyl, (C₁-C₇)-alkoxy, (C₁-C₇)-alkanoyloxy, carboxyl, (C₁-C₇)-alkoxycarbonyl, Cl, Br, amino, (C₁-C₇)-alkylamino, di-(C₁-C₇)-alkylamino, (C₁-C₅)-alkoxycarbonylamino, (C₇-C₁₅)-aralkoxycarbonylamino and 9-fluorenylmethyloxycarbonylamino, or represents (C₃-C₈)-cycloalkyl, (C₃-C₈)-cycloalkyl-(C₁-C₆)-alkyl, (C₆-C₁₄)-aryl which is optionally substituted by one or two identical or different radicals from the series comprising F, Cl, Br, I, hydroxyl, (C₁-C₇)-alkoxy, (C₁-C₇)-alkyl, (C₁-C₇)-alkoxycarbonyl, amino, anilino which is optionally substituted by up to 2 halogens, and trifluoromethyl, or represents (C₆-C₁₄)-aryl-(C₁-C₆)-alkyl in which the aryl moiety is optionally substituted by one or two identical or different radicals from the series comprising F, Cl, Br, I, hydroxyl, (C₁-C₇)-alkoxy, (C₁-C₇)-alkyl, (C₁-C₇)-alkoxycarbonyl, amino, (C₁-C₇)-alkylamino, di-(C₁-C₇)-alkylamino, carboxyl, carboxymethoxy, amino (C₁-C₇)-alkyl, (C₁-C₇)-alkylamino-(C₁-C₇)-alkyl, di-(C₁-C₇)-alkylamino-(C₁-C₇)-alkyl, (C₁-C₇)-alkoxycarbonylmethoxy, carbamoyl, sulfamoyl, (C₁-C₇)-alkoxysulfonyl, sulfo- and guanidinomethyl, or represents the radical of a 5- or 6-membered monocyclic, or 9- or 10-membered bicyclic, heteroaromatic compound which has at least 1 carbon atom, 1-4 nitrogen atoms and/or 1 sulfur or oxygen atom as ring members, and is optionally substituted by one or two identical or different radicals from the series comprising F, Cl, Br, hydroxyl, (C₁-C₇)-alkoxy, (C₁-C₇)-alkyl, (C₁-C₇)-alkoxycarbonyl, amino or trifluoromethyl,
R⁴ denotes a radical of the formula IV
-X-(CH₂)_{n'}-R⁷ (IV),
A denotes a radical, which is linked N-terminal with R¹ and C-terminal with B, of an amino acid from the series comprising phenylalanine, histidine, tyrosine, tryptophan, methionine, leucine, isoleucine, asparagine, aspartic acid, β-2-thienylalanine, norvaline, β-3-thienylalanine, β-2-furylalanine, β-3-furylalanine, lysine, ornithine, valine, alanine, 2,4-diaminobutyric acid, arginine, 4-chlorphenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert-butyltyrosine, phenylglycine, 1-naphthylalanine, 2-naphthylalanine, 4-nitrophenylalanine, β-2-benzo-[b]thienylalanine, β-3-benzo[b]thienylalanine, 2-fluorophenylalanine, 3-fluorophenylalanine, 4-fluorophenylalanine, norleucine, cysteine, S-methylcysteine, 1,2,3,4-tetrahydroisoquinoline-3-carboxylic acid, homophenylalanine, DOPA, O-dimethyldopa, 2-amino-4-(2-thienyl)butyric acid, 2-amino-4-(3-thienyl)butyric acid, 3-(2-thienyl)serine, (Z)-dehydrophenylalanine and (E)-dehydrophenylalanine,
B denotes a radical of an amino acid as defined under A,
R² denotes hydrogen, (C₁-C₁₀)-alkyl, (C₄-C₇)-cycloalkyl, (C₄-C₇)-cycloalkyl-(C₁-C₄)-alkyl, (C₆-C₁₄)-aryl or (C₆-C₁₄)-aryl-(C₁-C₄)-alkyl, and
R³ denotes hydrogen, (C₁-C₁₀)-alkyl, (C₆-C₁₄)-aryl or (C₆-C₁₄)-aryl-(C₁-C₄)-alkyl,
X is absent,
n' can be 0, 1, 2, 3 or 4,
R⁷ denotes heteroaryl which can also be partially hydrogenated, or a physiologically tolerated salt thereof, with the exception of a compound in which R² is butyl, R³ is H and R⁷ is 4-imidazole.

2. A compound of the formula I as claimed in claim 1, in which R¹ denotes (C₁-C₁₁)-alkanoyl, preferably n-decanoyl, formyl, acetyl, pivaloyl, isovaleryl or isobutyryl, optionally protected amino-(C₁-C₁₁)-alkanoyl, preferably 4-aminobutyryl, 5-aminopentanoyl, 6-aminohexanoyl, 4-N-tert-butoxycarbonylaminobutyryl, 5-N-tert-butoxycarbonylaminopentanoyl or 6-N-tert-butoxycarbonylaminohexanoyl, di-(C₁-C₇)-alkylamino-(C₂-C₁₁)-alkanoyl, preferably dimethylaminoacetyl, (C₄-C₉)-cycloalkylcarbonyl, preferably cyclopropylcarbonyl, cyclobutylcarbonyl, cyclopentylcarbonyl or cyclohexylcarbonyl, (C₆-C₁₀)-aryl-(C₂-C₁₁)-alkanoyl, preferably phenylacetyl, phenylpropanoyl or phenylbutanoyl, 2-(2,6-dichloroanilino)phenylacetyl or 2-(N-benzyl-2,6-dichloroanilino)phenylacetyl, or benzoyl which is optionally substituted by halogen, (C₁-C₇)-alkyl, (C₁-C₇)-alkoxy or (C₁-C₇)-alkoxycarbonyl, preferably 4-chlorobenzyoyl, 4-methylbenzoyl, 2-methoxycarbonylbenzoyl or 4-methoxybenzoyl, pyrolyl-2-carbonyl, pyridyl-3-carbonyl, benzenesulfonyl, (C₁-C₁₀)-alkoxycarbonyl, preferably methoxycarbonyl, ethoxycarbonyl or tert-butoxycarbonyl, (C₁-C₁₀)-alkoxycarbonyl substituted by halogen, preferably 2,2,2-trichloroethoxycarbonyl or 1,1-dimethyl-2,2,2-trichloroethoxycarbonyl, (C₆-C₁₄)-aryl-(C₁-C₆)-alkoxycarbonyl, preferably benzyloxycarbonyl or 9-fluorenylmethylcarbonyl, or a physiologically tolerated salt thereof.

3. A compound of the formula I as claimed in either or both of claims 1-2, in which R² denotes isobutyl, benzyl or cyclohexylmethyl, or a physiologically tolerated salt thereof.

4. A compound of the formula I as claimed in one or more of claims 1-3, in which R³ denotes hydrogen, isopropyl or isobutyl, or a physiologically tolerated salt thereof.

5. A process for the preparation of a compound of the formula I as claimed in any one of claims 1 to 4, which comprises coupling a fragment having a terminal carboxyl group, or a reactive derivative thereof, with an appropriate fragment having a free amino group, where appropriate eliminating (a) protective group(s) temporarily introduced to protect other functional groups, and, where appropriate, converting the resulting compound into its physiologically tolerated salt.

6. A pharmaceutical agent containing a compound as claimed in one of claims 1 to 4.

## Claims (Claims for the following Contracting State(s): AT, ES, GR)

1. A process for the preparation of a compound of the formula I 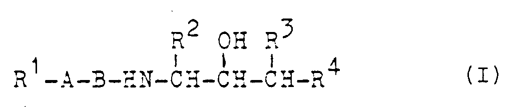 in which
R¹ denotes a radical of the formula II
R^{a}-W- (II)
in which W represents -CO-, -O-CO-, -SO₂- or -NH-CO-, and R^{a} represents hydrogen, (C₁-C₁₀)-alkyl which is optionally singly or doubly unsaturated and which is optionally substituted by up to 3 identical or different radicals from the series comprising hydroxyl, (C₁-C₇)-alkoxy, (C₁-C₇)-alkanoyloxy, carboxyl, (C₁-C₇)-alkoxycarbonyl, Cl, Br, amino, (C₁-C₇)-alkylamino, di-(C₁-C₇)-alkylamino, (C₁-C₅)-alkoxycarbonylamino, (C₇-C₁₅)-aralkoxycarbonylamino and 9-fluorenylmethyloxycarbonylamino, or represents (C₃-C₈)-cycloalkyl, (C₃-C₈)-cycloalkyl-(C₁-C₆)-alkyl, (C₆-C₁₄)-aryl which is optionally substituted by one or two identical or different radicals from the series comprising F, Cl, Br, I, hydroxyl, (C₁-C₇)-alkoxy, (C₁-C₇)-alkyl, (C₁-C₇)-alkoxycarbonyl, amino, anilino which is optionally substituted by up to 2 halogens, and trifluoromethyl, or represents (C₆-C₁₄)-aryl-(C₁-C₆)-alkyl in which the aryl moiety is optionally substituted by one or two identical or different radicals from the series comprising F, Cl, Br, I, hydroxyl, (C₁-C₇)-alkoxy, (C₁-C₇)-alkyl, (C₁-C₇)-alkoxycarbonyl, amino, (C₁-C₇)-alkylamino, di-(C₁-C₇)-alkylamino, carboxyl, carboxymethoxy, amino(C₁-C₇)-alkyl, (C₁-C₇)-alkylamino-(C₁-C₇)-alkyl, di-(C₁-C₇)-alkylamino-(C₁-C₇)-alkyl,(C₁-C₇)-alkoxycarbonylmethoxy, carbamoyl, sulfamoyl, (C₁-C₇)-alkoxysulfonyl, sulfo- and guanidinomethyl, or represents the radical of a 5- or 6-membered monocyclic, or 9- or 10-membered bicyclic, heteroaromatic compound which has at least 1 carbon atom, 1-4 nitrogen atoms and/or 1 sulfur or oxygen atom as ring members, and is optionally substituted by one or more identical or different radicals from the series comprising F, Cl, Br, hydroxyl, (C₁-C₇)-alkoxy, (C₁-C₇)-alkyl, (C₁-C₇)-alkoxycarbonyl, amino or trifluoromethyl,
R⁴ denotes a radical of the formula IV
-X-(CH₂)_{n'}-R⁷ (IV),
A denotes a radical, which is linked N-terminal with R¹ and C-terminal with B, of an amino acid from the series comprising phenylalanine, histidine, tyrosine, tryptophan, methionine, leucine, isoleucine, asparagine, aspartic acid, β-2-thienylalanine, norvaline, β-3-thienylalanine, β-2-furylalanine, β-3-furylalanine, lysine, ornithine, valine, alanine, 2,4-diaminobutyric acid, arginine, 4-chlorphenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert-butyltyrosine, phenylglycine, 1-naphthylalanine, 2-naphthylalanine, 4-nitrophenylalanine, β-2-benzo-[b]thienylalanine, β-3-benzo[b]thienylalanine, 2-fluorophenylalanine, 3-fluorophenylalanine, 4-fluorophenylalanine, norleucine, cysteine, S-methylcysteine, 1,2,3,4-tetrahydroisoquinoline-3-carboxylic acid, homophenylalanine, DOPA, O-dimethyldopa, 2-amino-4-(2-thienyl)butyric acid, 2-amino-4-(3-thienyl)butyric acid, 3-(2-thienyl)serine, (Z)-dehydrophenylalanine and (E)-dehydrophenylalanine,
B denotes a radical of an amino acid as defined under A,
R² denotes hydrogen, (C₁-C₁₀)-alkyl, (C₄-C₇)-cycloalkyl, (C₄-C₇)-cycloalkyl-(C₁-C₄)-alkyl, (C₆-C₁₄)-aryl or (C₆-C₁₄)-aryl-(C₁-C₄)-alkyl, and
R³ denotes hydrogen, (C₁-C₁₀)-alkyl, (C₆-C₁₄)-aryl or (C₆-C₁₄)-aryl-(C₁-C₄)-alkyl,
X is absent,
n' can be 0, 1, 2, 3 or 4,
R⁷ denotes heteroaryl which can also be partially hydrogenated, with the exception of a compound in which R² is butyl, R³ is H and R⁷ is 4-imidazole, or a physiologically tolerated salt thereof, which comprises coupling a fragment having a terminal carboxyl group, or a reactive derivative thereof, with an appropriate fragment having a free amino group, where appropriate eliminating (a) protective group(s) temporarily introduced to protect other functional groups, and, where appropriate, converting the resulting compound into its physiologically tolerated salt.

2. The process for the preparation of a compound of the formula I as claimed in claim 1, in which R¹ denotes (C₁-C₁₁)-alkanoyl, preferably n-decanoyl, formyl, acetyl, pivaloyl, isovaleryl or isobutyryl, optionally protected amino-(C₁-C₁₁)-alkanoyl, preferably 4-aminobutyryl, 5-aminopentanoyl, 6-aminohexanoyl, 4-N-tert-butoxycarbonylamino-butyryl, 5-N-tert-butoxycarbonylaminopentanoyl or 6-N-tert-butoxycarbonylaminohexanoyl, di-(C₁-C₇)-alkylamino-(C₂-C₁₁)-alkanoyl, preferably dimethylaminoacetyl, (C₄-C₉)-cycloalkylcarbonyl, preferably cyclopropylcarbonyl, cyclobutylcarbonyl, cyclopentylcarbonyl or cyclohexylcarbonyl, (C₆-C₁₀)-aryl-(C₂-C₁₁)-alkanoyl, preferably phenylacetyl, phenylpropanoyl or phenylbutanoyl, 2-(2,6-dichloroanilino)phenylacetyl or 2-(N-benzyl-2,6-dichloroanilino)phenylacetyl, or benzoyl which is optionally substituted by halogen, (C₁-C₇)-alkyl, (C₁-C₇)-alkoxy or (C₁-C₇)-alkoxycarbonyl, preferably 4-chlorobenzyoyl, 4-methylbenzoyl, 2-methoxycarbonylbenzoyl or 4-methoxybenzoyl, pyrolyl-2-carbonyl, pyridyl-3-carbonyl, benzenesulfonyl, (C₁-C₁₀)-alkoxycarbonyl, preferably methoxycarbonyl, ethoxycarbonyl or tert-butoxycarbonyl, (C₁-C₁₀)-alkoxycarbonyl substituted by halogen, preferably 2,2,2-trichloroethoxycarbonyl or 1,1-dimethyl-2,2,2-trichloroethoxycarbonyl, (C₆-C₁₄)-aryl-(C₁-C₆)-alkoxycarbonyl, preferably benzyloxycarbonyl or 9-fluorenylmethylcarbonyl, or a physiologically tolerated salt thereof.

3. The process for the preparation of a compound of the formula I as claimed in either or both of claims 1-2, in which R² denotes isobutyl, benzyl or cyclohexylmethyl, or of a physiologically tolerated salt thereof.

4. The process for the preparation of a compound of the formula I as claimed in one or more of claims 1-3, in which R³ denotes hydrogen, isopropyl or isobutyl, and its physiologically tolerated salts.

5. A process for the preparation of a pharmaceutical agent containing a compound of the formula I as claimed in one of claims 1-4, which comprises converting the latter and an excipient into a suitable form for administration.

## Revendications (Revendications pour l'(les) Etat(s) contractant(s) suivant(s): BE, CH, DE, FR, GB, IT, LI, LU, NL, SE)

1. Composé de formule I 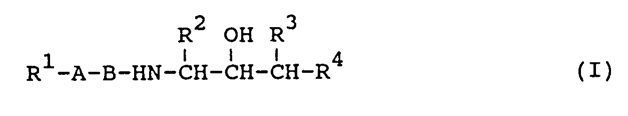 dans laquelle
R¹ représente un radical de formule II
R^{a}-W- (II)
dans laquelle W représente -CO-, -O-CO-, -SO₂- ou -NH-CO-, et R^{a} représente un atome d'hydrogène ou un groupe alkyle en C₁-C₁₀ qui est éventuellement une ou deux fois insaturé et qui est éventuellement substitué par jusqu'à trois substituants identiques ou différents, choisis parmi des radicaux hydroxy, alcoxy en C₁-C₇, alcanoyloxy en C₁-C₇, carboxy, alcoxy(C₁-C₇)-carbonyle, Cl, Br, amino, alkyl(C₁-C₇)-amino, dialkyl(C₁-C₇)-amino, alcoxy(C₁-C₅)-carbonylamino, aralcoxy(C₇-C₁₅)-carbonylamino et 9-fluorénylméthyloxycarbonylamino,
un groupe cycloalkyle en C₃-C₈, cycloalkyl(C₃-C₈)-alkyle-(C₁-C₆), un groupe aryle en C₆-C₁₄ qui est éventuellement substitué par un ou deux substituants, identiques ou différents, choisis parmi les atomes de F, Cl, Br, I, et des radicaux hydroxy, alcoxy en C₁-C₇, alkyle en C₁-C₇, alcoxy(C₁-C₇)-carbonyle, amino, un radical anilino éventuellement substitué par jusqu'à 2 atomes d'halogène et le radical trifluorométhyle,
un groupe aryl(C₆-C₁₄)-alkyle(C₁-C₆), dans lequel le fragment aryle est éventuellement substitué par un ou deux substituants, identiques ou différents, choisis parmi les atomes de F, Cl, Br, I, et des radicaux hydroxy, alcoxy en C₁-C₇, alkyle en C₁-C₇, alcoxy(C₁-C₇)-carbonyle, amino, alkyl(C₁-C₇)-amino, dialkyl(C₁-C₇)-amino, carboxy, carboxyméthoxy, amino-alkyle(C₁-C₇), alkyl(C₁-C₇)aminoalkyle(C₁-C₇), dialkyl(C₁-C₇)amino-alkyle(C₁-C₇), alcoxy-(C₁-C₇)carbonyl-méthoxy, carbamoyle, sulfamoyle, alcoxy-(C₁-C₇)sulfonyle, sulfo- et guanidinométhyle,
ou représente le reste d'un composé hétéroaromatique monocyclique à 5 ou 6 chaînons ou bicyclique à 9 ou 10 chaînons, comportant au moins 1 atome de carbone, 1-4 atomes d'azote et/ou 1 atome de soufre ou d'oxygène, en tant que chaînons formant le cycle, qui est éventuellement substitué par un ou deux substituants, identiques ou différents, choisis parmi les atomes de F, Cl, Br et des groupes hydroxy, alcoxy en C₁-C₇, alkyle en C₁-C₇, alcoxy(C₁-C₇)-carbonyle, amino ou trifluorométhyle,
R⁴ représente un radical de formule IV
-X-(CH₂)_{n'}-R⁷ (IV)
A représente un reste, lié à l'extrémité N-terminale à R¹ et à l'extrémité C-terminale à B, d'un aminoacide choisi parmi la phénylalanine, l'histidine, la tyrosine, le tryptophane, la méthionine, la leucine, l'isoleucine, l'asparagine, l'acide aspartique, la β-2-thiénylalanine, la norvaline, la β-3-thiénylalanine, la β-2-furylalanine, la β-3-furylalanine, la lysine, l'ornithine, la valine, l'alanine, l'acide 2,4-diaminobutyrique, l'arginine, la 4-chlorophénylalanine, la méthionine-sulfone, le méthionine-sulfoxyde, la 2-pyridylalanine, la 3-pyridylalanine, la cyclohexylalanine, la cyclohexylglycine, l'im-méthylhistidine, l'O-méthyltyrosine, l'O-benzyltyrosine, l'O-tert-butyltyrosine, la phénylglycine, la 1-naphtylalanine, la 2-naphtylalanine, la 4-nitrophénylalanine, la β-2-benzo[b]thiénylalanine, la β-3-benzo[b]thiénylalanine, la 2-fluorophénylalanine, la 3-fluorophénylalanine, la 4-fluorophénylalanine, la norleucine, la cystéine, la S-méthylcystéine, l'acide 1,2,3,4-tétrahydroisoquinoléine-3-carboxylique, l'homophénylalanine, la DOPA, l'O-diméthyldopa, l'acide 2-amino-4-(2-thiényl)-butyrique, l'acide 2-amino-4-(3-thiényl)-butyrique, la 3-(2-thiényl)-sérine, la (Z)-déhydrophénylalanine et la (E)-déhydrophénylalanine,
B représente un reste d'un aminoacide tel que défini en A,
R² représente un atome d'hydrogène ou un groupe alkyle en C₁-C₁₀, cycloalkyle en C₄-C₇, cycloalkyl(C₄-C₇)-alkyle-(C₁-C₄), aryle en C₆-C₁₄ ou aryl(C₆-C₁₄)-alkyle(C₁-C₄), et
R³ représente un atome d'hydrogène ou un groupe alkyle en C₁-C₁₀, aryle en C₆-C₁₄ ou aryl(C₆-C₁₄)-alkyle(C₁-C₄),
X est absent,
n' peut être 0, 1, 2, 3 ou 4,
R⁷ représente un groupe hétéroaryle qui peut être partiellement hydrogéné,
et sels physiologiquement acceptables de celui-ci, à l'exclusion des composés dans lesquels R² représente le groupe butyle, R³ = H et R⁷ représente le groupe 4-imidazole.

2. Composé de formule I selon la revendication 1, dans lequel R¹ représente un groupe alcanoyle en C₁-C₁₁, de préférence le groupe n-décanoyle, formyle, acétyle, pivaloyle, isovaléryle ou isobutyryle, un groupe aminoalcanoyle en C₆-C₁₁ éventuellement protégé, de préférence le groupe 4-aminobutyryle, 5-aminopentanoyle, 6-aminohexanoyle, 4-N-tert-butoxycarbonylaminobutyryle, 5-N-tert-butoxycarbonylaminopentanoyle ou 6-N-tert-butoxycarbonylaminohexanoyle, un groupe dialkyl(C₁-C₇)amino-alcanoyle(C₂-C₁₁), de préférence le groupe diméthylaminoacétyle, un groupe cycloalkyl(C₄-C₉)-carbonyle, de préférence le groupe cyclopropylcarbonyle, cyclobutylcarbonyle, cyclopentylcarbonyle ou cyclohexylcarbonyle, un groupe aryl(C₆-C₁₀)-alcanoyle(C₂-C₁₁), de préférence le groupe phénylacétyle, phénylpropanoyle ou phénylbutanoyle, le groupe 2-(2,6-dichloranilino)-phénylacétyle, 2-(N-benzyl-2,6-dichloranilino)-phénylacétyle, un groupe benzoyle éventuellement substitué par un atome d'halogène ou par un groupe alkyle en C₁-C₇, alcoxy en C₁-C₇, alcoxy(C₁-C₇)-carbonyle, de préférence le groupe 4-chlorobenzoyle, 4-méthylbenzoyle, 2-méthoxycarbonylbenzoyle ou 4-méthoxybenzoyle, le groupe pyrrolyl-2-carbonyle, pyridyl-3-carbonyle, benzènesulfonyle, alcoxy(C₁-C₁₀)-carbonyle, de préférence le groupe méthoxycarbonyle, éthoxycarbonyle ou tert-butoxycarbonyle, un groupe alcoxy(C₁-C₁₀)-carbonyle substitué par un atome d'halogène, de préférence le groupe 2,2,2-trichloréthoxycarbonyle ou 1,1-diméthyl-2,2,2-trichloréthoxycarbonyle, un groupe aryl(C₆-C₁₄)-alcoxy(C₁-C₆)-carbonyle, de préférence le groupe benzyloxycarbonyle ou 9-fluorénylméthylcarbonyle, et sels physiologiquement acceptables de celui-ci.

3. Composé de formule I selon une ou plusieurs des revendications 1 et 2, dans lequel R² représente le groupe isobutyle, benzyle ou cyclohexylméthyle, et sels physiologiquement acceptables de celui-ci.

4. Composé de formule I selon une ou plusieurs des revendications 1 à 3, dans lequel R³ représente un atome d'hydrogène ou le groupe isopropyle ou isobutyle, et sels physiologiquement acceptables de celui-ci.

5. Procédé pour la préparation d'un composé de formule I selon l'une des revendications 1 à 4, caractérisé en ce que l'on assemble un fragment à groupe carboxy terminal ou un dérivé réactif de celui-ci avec un fragment correspondant à groupe amino libre, éventuellement on élimine un(des) groupe(s) protecteur(s) introduit(s) temporairement pour la protection d'autres groupes fonctionnels, et éventuellement on convertit le composé ainsi obtenu en un de ses sels physiologiquement acceptables.

6. Produit pharmaceutique contenant un composé selon l'une des revendications 1 à 4.

## Revendications (Revendications pour l'(les) Etat(s) contractant(s) suivant(s): AT, ES, GR)

1. Procédé pour la préparation d'un composé de formule I 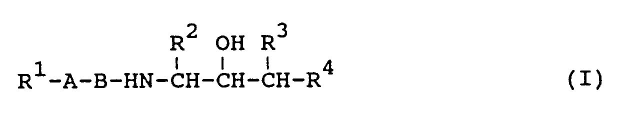 dans laquelle
R¹ représente un radical de formule II
R^{a}-W- (II)
dans laquelle W représente -CO-, -O-CO-, -SO₂- ou -NH-CO-, et R^{a} représente un atome d'hydrogène ou un groupe alkyle en C₁-C₁₀ qui est éventuellement une ou deux fois insaturé et qui est éventuellement substitué par jusqu'à trois substituants identiques ou différents, choisis parmi des radicaux hydroxy, alcoxy en C₁-C₇, alcanoyloxy en C₁-C₇, carboxy, alcoxy(C₁-C₇)-carbonyle, Cl, Br, amino, alkyl(C₁-C₇)-amino, dialkyl(C₁-C₇)-amino, alcoxy(C₁-C₅)-carbonylamino, aralcoxy(C₇-C₁₅)-carbonylamino et 9-fluorénylméthyloxycarbonylamino,
un groupe cycloalkyle en C₃-C₈, cycloalkyl(C₃-C₈)-alkyle-(C₁-C₆), un groupe aryle en C₆-C₁₄ qui est éventuellement substitué par un ou deux substituants, identiques ou différents, choisis parmi les atomes de F, Cl, Br, I, et des radicaux hydroxy, alcoxy en C₁-C₇, alkyle en C₁-C₇, alcoxy(C₁-C₇)-carbonyle, amino, un radical anilino éventuellement substitué par jusqu'à 2 atomes d'halogène et le radical trifluorométhyle,
un groupe aryl(C₆-C₁₄)-alkyle(C₁-C₆), dans lequel le fragment aryle est éventuellement substitué par un ou deux substituants, identiques ou différents, choisis parmi les atomes de F, Cl, Br, I, et des radicaux hydroxy, alcoxy en C₁-C₇, alkyle en C₁-C₇, alcoxy(C₁-C₇)-carbonyle, amino, alkyl(C₁-C₇)-amino, dialkyl(C₁-C₇)-amino, carboxy, carboxyméthoxy, amino-alkyle(C₁-C₇), alkyl(C₁-C₇)aminoalkyle(C₁-C₇), dialkyl(C₁-C₇)amino-alkyle(C₁-C₇), alcoxy(C₁-C₇)carbonyl-méthoxy, carbamoyle, sulfamoyle, alcoxy(C₁-C₇)sulfonyle, sulfo- et guanidinométhyle,
ou représente le reste d'un composé hétéroaromatique monocyclique à 5 ou 6 chaînons ou bicyclique à 9 ou 10 chaînons, comportant au moins 1 atome de carbone, 1-4 atomes d'azote et/ou 1 atome de soufre ou d'oxygène, en tant que chaînons formant le cycle, qui est éventuellement substitué par un ou deux substituants, identiques ou différents, choisis parmi les atomes de F, Cl, Br et des groupes hydroxy, alcoxy en C₁-C₇, alkyle en C₁-C₇, alcoxy(C₁-C₇)-carbonyle, amino ou trifluorométhyle,
R⁴ représente un radical de formule IV
-X-(CH₂)_{n'}-R⁷ (IV)
A représente un reste, lié à l'extrémité N-terminale à R¹ et à l'extrémité C-terminale à B, d'un aminoacide choisi parmi la phénylalanine, l'histidine, la tyrosine, le tryptophane, la méthionine, la leucine, l'isoleucine, l'asparagine, l'acide aspartique, la β-2-thiénylalanine, l'alanine, la norvaline, la β-3-thiénylaline, la β-2-furylalanine, la β-3-furylalanine, la lysine, l'ornithine, la valine, l'acide 2,4-diaminobutyrique, l'arginine, la 4-chlorophénylalanine, la méthionine-sulfone, le méthionine-sulfoxyde, la 2-pyridylalanine, la 3-pyridylalanine, la cyclohexylalanine, la cyclohexylglycine, l'im-méthylhistidine, l'O-méthyltyrosine, l'O-benzyltyrosine, l'O-tert-butyltyrosine, la phénylglycine, la naphtylalanine, la 2-naphtylalanine, la 4-nitrophénylalanine, la β-2-benzo[b]thiénylalanine, la β-3-benzo[b]-thiénylalanine, la 2-fluorophénylalanine, la 3-fluorophénylalanine, la 4-fluorophénylalanine, la norleucine, l'acide 1,2,3,4-tétrahydroisoquinoléine-3-carboxylique, l'homophénylalanine, la DOPA, l'O-diméthyldopa, l'acide 2-amino-4-(2-thiényl)-butyrique, l'acide 2-amino-4-(3-thiényl)-butyrique, la 3-(2-thiényl)-sérine, la (Z)-déhydrophénylalanine et la (E)-déhydrophénylalanine,
B représente un reste d'un aminoacide tel que défini en A,
R² représente un atome d'hydrogène ou un groupe alkyle en C₁-C₁₀, cycloalkyle en C₄-C₇, cycloalkyl(C₄-C₇)-alkyle-(C₁-C₄), aryle en C₆-C₁₄ ou aryl(C₆-C₁₄)-alkyle(C₁-C₄), et
R³ représente un atome d'hydrogène ou un groupe alkyle en C₁-C₁₀, aryle en C₆-C₁₄ ou aryl(C₆-C₁₄)-alkyle(C₁-C₄),
X est absent,
n' peut être 0, 1, 2, 3 ou 4,
R⁷ représente un groupe hétéroaryle qui peut être partiellement hydrogéné,
à l'exclusion des composés dans lesquels R² représente le groupe butyle, R³ = H et R⁷ représente le groupe 4-imidazole et de ses sels physiologiquement acceptables, caractérisé en ce que l'on assemble un fragment à groupe carboxy terminal ou un dérivé réactif de celui-ci avec un fragment correspondant à groupe amino libre, éventuellement on élimine un(des) groupe(s) protecteur(s) introduit(s) temporairement pour la protection d'autres groupes fonctionnels, et éventuellement on convertit le composé ainsi obtenu en un de ses sels physiologiquement acceptables.

2. Procédé pour la préparation d'un composé de formule I selon la revendication 1, dans lequel R¹ représente un groupe alcanoyle en C₁-C₁₁, de préférence le groupe n-décanoyle, formyle, acétyle, pivaloyle, isovaléryle ou isobutyryle, un groupe amino-alcanoyle en C₆-C₁₁ éventuellement protégé, de préférence le groupe 4-aminobutyryle, 5-aminopentanoyle, 6-aminohexanoyle, 4-N-tert-butoxycarbonylaminobutyryle, 5-N-tert-butoxycarbonylaminopentanoyle ou 6-N-tert-butoxycarbonylaminohexanoyle, un groupe dialkyl(C₁-C₇)amino-alcanoyle(C₂-C₁₁), de préférence le groupe diméthylaminoacétyle, un groupe cycloalkyl-(C₄-C₉)-carbonyle, de préférence le groupe cyclopropylcarbonyle, cyclobutylcarbonyle, cyclopentyl-carbonyle ou cyclohexylcarbonyle, un groupe aryl(C₆-C₁₀)-alcanoyle(C₂-C₁₁), de préférence le groupe phénylacétyle, phénylpropanoyle ou phénylbutanoyle, le groupe 2-(2,6-dichloranilino)-phénylacétyle, 2-(N-benzyl-2,6-dichloranilino)-phénylacétyle, un groupe benzoyle éventuellement substitué par un atome d'halogène ou par un groupe alkyle en C₁-C₇, alcoxy en C₁-C₇, alcoxy(C₁-C₇)-carbonyle, de préférence le groupe 4-chlorobenzoyle, 4-méthylbenzoyle, 2-méthoxycarbonylbenzoyle ou 4-méthoxybenzoyle, le groupe pyrrolyl-2-carbonyle, pyridyl-3-carbonyle, benzènesulfonyle, alcoxy-(C₁-C₁₀)-carbonyle, de préférence le groupe méthoxycarbonyle, éthoxycarbonyle ou tert-butoxycarbonyle, un groupe alcoxy(C₁-C₁₀)-carbonyle substitué par un atome d'halogène, de préférence le groupe 2,2,2-trichloréthoxycarbonyle ou 1,1-diméthyl-2,2,2-trichloréthoxycarbonyle, un groupe aryl(C₆-C₁₄)-alcoxy(C₁-C₆)-carbonyle, de préférence le groupe benzyloxycarbonyle ou 9-fluorénylméthylcarbonyle, et de ses sels physiologiquement acceptables.

3. Procédé pour la préparation d'un composé de formule I selon une ou plusieurs des revendications 1 et 2, dans lequel R² représente le groupe isobutyle, benzyle ou cyclohexylméthyle, et de ses sels physiologiquement acceptables.

4. Procédé pour la préparation d'un composé de formule I selon une ou plusieurs des revendications 1 à 3, dans lequel R³ représente un atome d'hydrogène ou le groupe isopropyle ou isobutyle, et de ses sels physiologiquement acceptables.

5. Procédé pour la fabrication d'un produit pharmaceutique contenant un composé de formule I selon l'une des revendications 1 à 4, caractérisé en ce qu'on le met, avec un véhicule, sous une forme d'administration appropriée.
